(19) Europäisches Patentamt
European Patent Office
Office européen des brevets

(11) Veröffentlichungsnummer: **0 252 441 B1**

(12) **EUROPÄISCHE PATENTSCHRIFT**

(45) Veröffentlichungstag der Patentschrift: **30.10.91**

(51) Int. Cl.$^5$: **C07C 215/02**, A61K 7/08

(21) Anmeldenummer: **87109535.2**

(22) Anmeldetag: **02.07.87**

# (54) Neue quartäre Ammoniumverbindungen und deren Verwendung.

(30) Priorität: **10.07.86 DE 3623215**

(43) Veröffentlichungstag der Anmeldung:
**13.01.88 Patentblatt 88/02**

(45) Bekanntmachung des Hinweises auf die Patenterteilung:
**30.10.91 Patentblatt 91/44**

(84) Benannte Vertragsstaaten:
**DE**

(56) Entgegenhaltungen:
**EP-A- 0 022 562**
**CH-A- 415 964**
**GB-A- 1 087 413**

(73) Patentinhaber: **Henkel Kommanditgesellschaft auf Aktien**
**Postfach 1100 Henkelstrasse 67**
**W-4000 Düsseldorf-Holthausen(DE)**

(72) Erfinder: **Lange, Fritz, Dr.**
**Baderweg 82**
**W-4300 Essen(DE)**
Erfinder: **Busch, Peter, Dr.**
**Gottfried-August-Bürger-Strasse 10**
**W-4006 Erkrath(DE)**
Erfinder: **Thiele, Klaus**
**Rügenweg 5**
**W-4018 Langenfeld(DE)**

EP 0 252 441 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Gegenstand der Erfindung sind neue quartäre Ammoniumverbindungen und deren Verwendung als haarkonditionierende und die Kämmbarkeit verbessernde Zusätze in haarkosmetischen Mitteln.

Kationische Tenside vom Typ der quartären Ammoniumverbindungen werden in der Haarkosmetik als avivierende und konditonierende Wirkstoffe zur Verbesserung der Kämmbarkeit, der Fülle und des Griffs der Haare und zur Senkung der statischen Aufladbarkeit der Haare eingesetzt. Die Produkte werden meist in Haarnachbehandlungsmitteln verwendet, die nach der Haarwäsche dem Haar wieder günstige kaarkosmetische Eigenschaften verleihen sollen. Sie eignen sich aber wenig als Zusatzmittel zu Haarwaschmitteln, um gleichzeitig mit der Wäsche einen gewissen konditionierenden Effekt zu erzielen, da die meisten bekannten quartären Ammoniumverbindungen mit den in Shampoos gebräuchlichen schaumstarken anionischen Tensiden, in den für eine ausreichende konditionierende Wirkung erforderlichen Konzentrationen nicht verträglich sind, sondern schwer wasserlösliche und kosmetisch unwirksame Niederschläge bilden. Quartäre Ammoniumverbindungen, die mit Aniontensiden besser verträglich sind, weisen meist eine unbefriedigende konditionierende Wirkung auf.

Wasserlösliche kationische Polymere sind zwar meist mit anionischen Tensiden verträglich, haben aber andere Nachteile, z.B. Akkumulation auf dem Haar nach mehrmaliger Behandlung und die unzureichende Verminderung der statischen Aufladbarkeit des trockenen Haars.

Es bestand daher die Aufgabe, quartäre Ammoniumverbindungen zu finden, die starke avivierende und konditionierende Eigenschaften auf menschlichem Haar besitzen und die auch in Haarwaschmittel (Shampoos) auf Basis schaumstarker anionischer Tenside in Konzentrationen eingearbeitet werden können, die für eine befriedigende kosmetische Wirkung ausreichend sind ohne daß Trübungen und Ausfällungen auftreten.

Es wurden neue quartäre Ammoniumverbindungen gefunden, welche die vorstehend genannten Anforderungen in hohem Maße erfüllen. Die neuen quartären Ammoniumverbindungen sind gekennzeichnet durch die Formel I

(I) 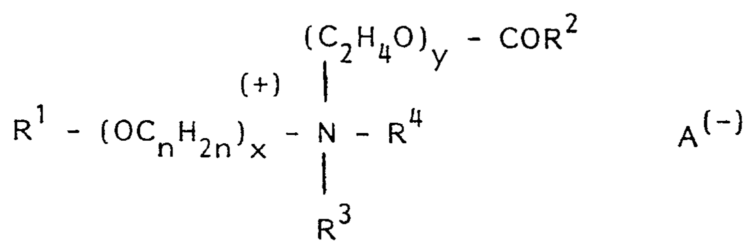

in der $R^1$ eine Alkylgruppe mit 8 bis 22 C-Atomen, $R^2$ eine Alkylgruppe mit 7 bis 21 C-Atomen, $R^3$ eine Alkylgruppe mit 1 bis 4 C-Atomen, eine Gruppe $-(C_2H_4O)_z-H$ oder $-(C_2H_4O)_z-COR^2$, $R^4$ eine Alkylgruppe mit 1 bis 4 C-Atomen oder eine Benzylgruppe, n = 2 oder 3, x, y und z Zahlen von 1 bis 10 und A ein Chlorid-, Bromid- oder ein Anion der Formel $R^5OSO_3^{(-)}$ ist, worin $R^5$ eine Alkylgruppe mit 1 bis 4 C-Atomen ist, darstellt.

Die Herstellung der neuen quartären Ammoniumverbindungen erfolgt ausgehend von Etheraminen der allgemeinen Formel II

(II) 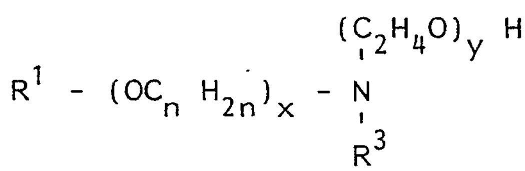

in der $R^3$ eine Alkylgruppe mit 1 bis 4 C-Atomen oder eine Gruppe $-(C_2H_4O)_z-H$ darstellt und $R^1$, n, x, y und z die für die Formel I angegebene Bedeutung haben, durch Veresterung mit einer Fettsäure der Formel $R^2$ - COOH, in der $R^2$ die für Formel I angegebene Bedeutung hat, unter an sich bekannten Reaktionsbedingungen und Quaternierung des erhaltenen Produktes mit einem Alkylhalogenid oder einem Dialkylsulfat der Formel $R^4$ A, in der $R^4$, A und n die für Formel I angegebene Bedeutung haben.

Verbindungen gemäß Formel I, bei denen der Parameter x allerdings die Bedeutung 0 hat, sind aus EP-A-22562 als Wäscheweichspülmittel bekannt. Hinweise auf die Eignung dieser Verbindungen zur Erzielung avivierender und konditionierender Eigenschaften auf menschlichem Haar sind dieser Druckschrift nicht zu entnehmen.

Etheramine der Formel II sind literaturbekannt, z.B. aus GB-A-1.087.413 und aus EP-A-102 140. Nach dem dort beschriebenen Herstellverfahren werden solche Etheramine ausgehend von primären und sekundären Aminen durch Alkylierung am Stickstoffatom, z.B. mit Alkylpolyglycolethersulfaten hergestellt. Ein weiteres Verfahren, welches Gegenstand der deutschen Patentanmeldung P 35 04 242.7 ist, geht aus von tertiären Alkanolaminen und führt durch Alkylierung an der Hydroxylgruppe, z.B. mit Alkylsulfaten oder Alkylpolyglycolethersulfaten zu Etheraminen der Formel II.

Verbindungen der Formel II, in welcher y (und/oder z) Werte von mehr als 1 aufweisen, können dadurch erhalten werden, daß man Etheramine der Formel II, worin y (und/oder z) = 1 ist mit (y - 1) + (z - 1) Mol Ethylenoxid nach dem dafür bekannten Verfahren umsetzt. Auf diese Weise läßt sich die Hydrophilie der erfindungsgemäßen quartären Ammoniumverbindungen in weitem Umfang einstellen.

Die Veresterung der Etheramine der Formel II mit Fettsäure verläuft nach allgemein bekannten Veresterungsverfahren, z.B. in Gegenwart von typischen basischen Veresterungskatalysatoren, bevorzugt von Zinnpulver oder Zinnoxid. Als Fettsäuren können alle in natürlichen Fetten vorkommenden Fettsäuren wie z.B. n-Octansäure (Caprylsäure), n-Decansäure (Caprinsäure), n-Do-decansäure, (Laurinsäure), n-Tetradecansäure (Myristinsäure), n-Hexadecansäure (Palmitinsäure), n-Octadecansäure (Stearinsäure), Ölsäure, Linolsäure, Arachinsäure, Erucasäure oder Behensäure eingesetzt werden. Geeignet sind auch synthetische und gegebenenfalls verzweigte Fettsäuren mit 8 bis 22 C-Atomen und Mischungen einzelner der genannten Fettsäuren. Die Veresterung wird im allgemeinen mit äquimolaren Mengen der Fettsäure, bezogen auf die freien Hydroxylgruppen, durchgeführt. Für den Fall, daß $R^3$ eine Gruppe $-(C_2H_4O)_z-H$ ist, können 1 bis 2 Mol Fettsäure für die Veresterung eingesetzt werden.

Die Quaternierung erfolgt ebenfalls nach bekannten Verfahren. Als Quaternierungsmittel eignen sich Alkylchloride und Alkylbromide sowie Dialkylsulfate mit 1 bis 4 C-Atomen in der Alkylgruppe sowie Benzylchlorid oder Benzylbromid.

Die erfindungsgemäßen quartären Ammoniumverbindungen haben oberflächenaktive Eigenschaften und wie alle quartären Ammoniumtenside sind sie substantiv, d.h. sie ziehen auf Faseroberflächen auf und zeigen dort antistatische und avivierende Eigenschaften. Darüber hinaus lassen sie sich in wässrigen Tensidlösungen klar solubilisieren. Überraschenderweise gelingt es, die erfindungsgemäßen quartären Ammoniumverbindungen auch in wässrigen Lösungen anionischer Tenside klar oder nur schwach opak zu solubilisieren ohne daß es zur Bildung schwerlöslicher Elektroneutralsalze kommt. Diese Eigenschaft macht die erfindungsgemäßen quartären Ammoniumverbindungen besonders geeignet für die Verwendung als konditionierende und avivierende Wirkstoffe in haarkosmetischen Mitteln.

Unter haarkosmetischen Mitteln werden alle Zubereitungen auf wässriger und wässrig-alkoholischer Basis verstanden, die zur pflegenden und dekorativen Behandlung des menschlichen Haares geeignet sind wie z.B. Haarwässer, Haarlotionen, Haarkurmittel, Haarfestiger, Haarfärbemittel, Haarwellmittel, Haarnachbehandlungsmittel, Haarspülmittel und Haarwaschmittel (Shampoos).

Eine besonders bevorzugte Ausführungsform der Erfindung sind wässrige Shampoos mit einem Gehalt von 5 bis 25 Gewichtsprozent anionischer Sulfat- und/oder Sulfonattenside und 0,1 bis 5 Gewichtsprozent quartärer Ammoniumverbindungen der Formel 1. Unter anionischen Sulfat- oder Sulfonattensiden werden dabei Alkali- und/oder Mono-, Di- oder Trialkanolaminsalze, mit 2- oder 3 C-Atomen in der Alkanolgruppe, von oberflächenaktiven Stoffen verstanden, die im Molekül eine ganz oder vorwiegend lineare Alkylgruppe mit 10 bis 16 C-Atomen und eine anionische $-SO_3^{(-)}$-oder $-OSO_3^{(-)}$-Gruppe enthalten. Bevorzugte Beispiele solcher Sulfat- oder Sulfonattenside sind Fettalkoholsulfate, Fettalkoholpolyglycolethersulfate (mit 1 bis 12 Glycolethergruppen), sekundäre Alkansulfonate und Alphaolefinsulfonate.

Bevorzugt geeignet sind erfindungsgemäße quartäre Ammoniumverbindungen der Formel I, worin y und z = 1, $R^4$ eine Methylgruppe und A ein Chloridanion ist.

## Beispiele

1. Kokos($C_{12}$-$C_{18}$)-alkyl-poly(6)-oxyethyl-di-(2-lauroyloxyethyl)-methyl-ammoniumchlorid.

1.1 Veresterung

800 g (1,183 Mol) Kokos ($C_{12}$-$C_{18}$)-alkyl-poly(6)oxyethyl-di-hydroxyethylamin (Aminzahl 83, hergestellt aus Kokos ($C_{12}$-$C_{18}$)-alkyl + 6 EO-sulfat, Na-Salz und Diethanolamin) und 472,2 g (2,366 Mol) Laurinsäure wurden in Gegenwart von 20 g Zinnpulver auf 220° C am Wasserabscheider erwärmt und 2 Stunden bis zur Beendigung der Wasserabspaltung bei dieser Temperatur gerührt. Dann wurde das Reaktionsgemisch von dem Zinnpulver abfiltriert. Es wurde klares, viskoses braungelbes Öl mit

einer Rest-Säurezahl von 1,38 erhalten.

1.2 Quaternierung

600 g des Reaktionsproduktes nach 1.1 wurden mit Methylchlorid im Druckautoklaven bei 90 °C und 10 bar zur Umsetzung gebracht. Nach etwa 5 Stunden wurde kein Methylchlorid mehr aufgenommen und die Aminzahl des Reaktionsgemisches war auf 2,2 abgefallen. Es wurde ein braunes wachsartiges Produkt erhalten.

2. Kokos($C_{12}$-$C_{18}$)-alkyl-poly(6)oxyethyl-2-lauroyloxyethyl-2-hydroxyethyl-methyl-ammoniumchlorid

2.1 Veresterung

1000 g (1,48 Mol) Kokos ($C_{12}$-$C_{18}$)-alkyl-poly(6)oxyethyl-dihydroxyethylamin (Aminzahl 83, hergestellt aus Kokos ($C_{12}$-$C_{18}$-alkyl + 6 EO-sulfat, Na-Salz und Diethanolamin) und 295 g (1,48 Mol) Laurinsäure wurden in Gegenwart von 20 g Zinnpulver auf 220 °C am Wasserabscheider erwärmt und 2 Stunden bis zur Beendigung der Wasserabspaltung bei dieser Temperatur gerührt. Dann wurde das Reaktionsgemisch über Celite von dem Zinnpulver abfiltriert. Es wurde ein klares, gelbbraunes viskoses Öl mit einer Rest-Säurezahl von 1,0 erhalten.

2.2 Quaternierung

900 g des Reaktionsproduktes nach 2.1 wurden mit Methylchlorid im Druckautoklaven bei 80 °C und 5 bar zur Umsetzung gebracht bis kein Methylchlorid mehr aufgenommen wurde und nach etwa 8 Stunden die Aminzahl auf 2,4 abgefallen war. Es wurde ein braunes, wachsartiges Produkt erhalten.

3. Lauryl-myristyl-poly(3)oxyethyl-di(2-lauroyloxyethyl)-methyl-ammoniumchlorid

3.1 Alkylierung

968 g (1,67 Mol) Fettalkohol $C_{12}$-$C_{14}$ (7 : 3) 2 EO-sulfat-Na-Salz (65,8 %ige wässrige Lösung) wurden mit 300,7 g (2,018 Mol) Triethanolamin zusammengegeben und im Rotationsverdampfer bei 100 °C im Wasserstrahlvakuum weitgehend entwässert. Nach Spülung des Reaktionsgefäßes mit Stickstoff wurden 80,7 g (2,018 Mol) Natriumhydroxid zugesetzt, nochmals mit Stickstoff gespült und das Reaktionsgemisch 3 Stunden auf 200 °C erhitzt. Nach dem Abkühlen auf 20 °C wurde der Ansatz mehrmals mit gesättigter Kochsalzlösung ausgewaschen und am Rotationsverdampfer unter Vakuum und Erwärmen auf 100 °C getrocknet. Ausgefallenes Natriumsulfat wurd abfiltriert. Es wurden 565 g eines gelbbraunen Öls mit einer Aminzahl von 111,4 (Lauryl-myristyl-poly(3)-oxyethyl-dihydroxyethylamin) erhalten.

3.2 Veresterung

240 g dieses Produktes (0,483 Mol) und 193,3 g Laurinsäure (0,966 Mol) wurden in Gegenwart von 4,5 g Zinnpulver auf 170 °C am Wasserabscheider erhitzt und 3 Stunden bis zur Beendigung der Wasserabspaltung bei dieser Temperatur gerührt. Dann wurde das Reaktionsgemisch von dem Zinnpulver abfiltriert. Es wurde ein klares, viskoses, braungelbes Öl in einer Menge von 404 g (Säurezahl = 0) erhalten.

3.3 Quaternierung

300 g des Reaktionsproduktes nach 3.2 wurden mit Methylchlorid im Druckautoklaven bei 90 °C und 10 bar zur Umsetzung gebracht. Nach 3 Stunden wurde die Temperatur auf 110 °C erhöht, nach weiteren 3 Stunden wurde kein Methylenchlorid mehr aufgenommen. Die Aminzahl des Reaktionsgemisches betrug 10. Es wurde ein braunes wachsartiges Produkt erhalten.

4. Haarkosmetische Prüfung

Die erfindungsgemäßen quartären Ammoniumverbindungen gemäß Beispiel 1 und 2 wurden in Rezepturen 5.2 und 5.3 gemäß Tabelle eingearbeitet und gegenüber einem Standard-Shampoo (Rezeptur 5.1) ohne quartäre Ammoniumverbindung sowie einem Vergleichsshampoo mit Cetyltrimethylammoniumchlorid (Rezeptur 5.4) anwendungstechnisch auf die Naßkämmbarkeit verbessernde Wirkung geprüft.

Messung der Naßkämmbarkeit (Laborprüfung)

Es wurden standardisierte, unter definierten Bedingungen durch Blondierung und Kaltwelle vorgeschädigte Haarsträhnen verwendet. Diese wurden mit den in Tabelle angegebenen Shampoos in einer Menge von 1 g pro g Haar in handwarmem Wasser shampooniert und mit klarem Wasser nachgespült. Die Messung der Naßkämmbarkeit erfolgte durch eine Messung des Kämmwiderstandes, d.h. der Kraft, die erforderlich ist, um einen Kamm durch ein Haarbüschel zu ziehen. Dabei wurde eine modifizierte Zug-Prüfmaschine des Typs 1402 der Fa. Zwick (Einsingen in Ulm/Donau) verwendet. Die Prüfungsanordnung ist beschrieben in "Riechstoffe, Aromen, Kosmetika" Nr. 12, (1977), Seite 325, Spalte 2 und 3.

Um die Fehler möglichst gering zu halten, wurden 15-fache Bestimmungen des Kämmwiderstandes mit jedem der zu prüfenden Shampoos durchgeführt und die Mittelwerte gebildet. Die gemessenen Kämmwiderstandsmittelwerte wurden in Prozent des Standards angegeben. Der Standard wurde bestimmt nach

Shampoonieren mit dem Shampoo 1 ohne quartäre Ammoniumverbindung und Nachspülen mit klarem Wasser.

Die Ergebnisse der Prüfung sind der Tabelle zu entnehmen.

Tabelle

| Prüfshampoos | 1 | 2 | 3 | 4 |
|---|---|---|---|---|
| Fettalkohol ($C_{12-14}$) + 2 EO-sulfat-Na-Salz (28 %ig) | 50 | 50 | 50 | 50 |
| QAV Beispiel 1 | - | 2 | - | - |
| QAV Beispiel 2 | - | - | 2 | - |
| Cetyltrimethylammoniumchlorid | - | - | - | 2 |
| Wasser (vollentsalzt) | 50 | 48 | 48 | 48 |
| Aussehen bei 20 °C | klar | klar | klar | trüb (Bodensatzbildung) |
| Naßkämmbarkeit (% Kämmwiderstand) | 100 | 70 | 93 | 88 |

**Patentansprüche**

1. Quartäre Ammoniumverbindungen der Formel I

(I) 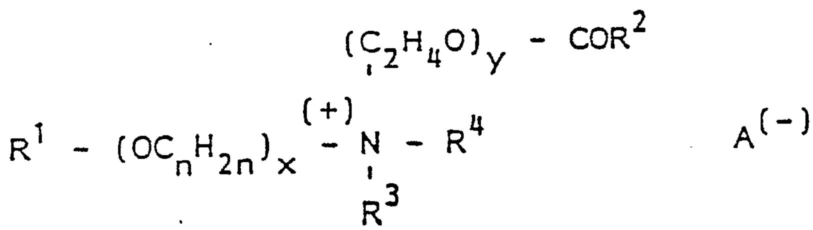

in der $R^1$ eine Alkylgruppe mit 8 bis 22 C-Atomen, $R^2$ eine Alkylgruppe mit 7 bis 21 C-Atomen, $R^3$ eine Alkylgruppe mit 1 bis 4 C-Atomen, eine Gruppe $-(C_2H_4O)_Z-H$ oder $-(C_2H_4O)_Z-COR^2$, $R^4$ eine Alkylgruppe mit 1 bis 4 C-Atomen oder eine Benzylgruppe, n = 2 oder 3, x, y und z Zahlen von 1 bis 10 und A ein Chlorid-, Bromid- oder ein Anion der Formel $R^5OSO_3^{(-)}$ ist, worin $R^5$ eine Alkylgruppe mit 1 bis 4 C-Atomen ist, darstellt.

(I) 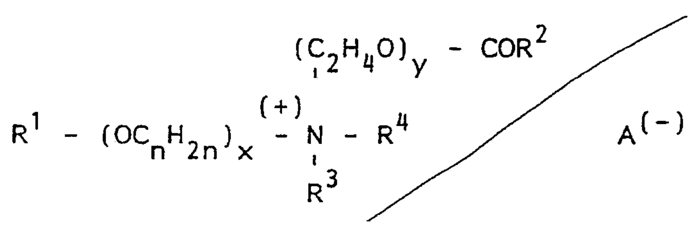

2. Quartäre Ammoniumverbindungen gemäß Anspruch 1, dadurch gekennzeichnet, daß y und z = 1, $R^4$ eine Methylgruppe und A ein Chloridanion ist.

3. Verfahren zur Herstellung quartärer Ammoniumverbindungen der Formel I gemäß Anspruch 1 oder 2, dadurch gekennzeichnet, daß man Etheramine der Formel II,

(II) 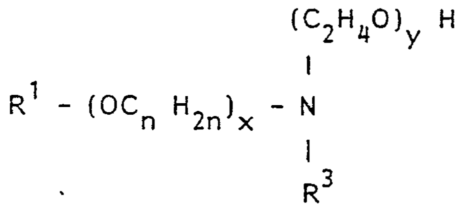

mit einer Fettsäure der Formel $R^2$- COOH unter an sich bekannten Reaktionsbedingungen verestert, das erhaltene Produkt mit einem Alkylhalogenid oder einem Dialkylsulfat der Formel $R^4$ A quaterniert.

4. Verwendung der quartären Ammoniumverbindungen der Formel I nach Anspruch 1 oder 2 als konditionierende und avivierende Wirkstoffe in haarkosmetischen Zubereitungen.

**Claims**

1. Quaternary ammonium compounds corresponding to formula I

(I) 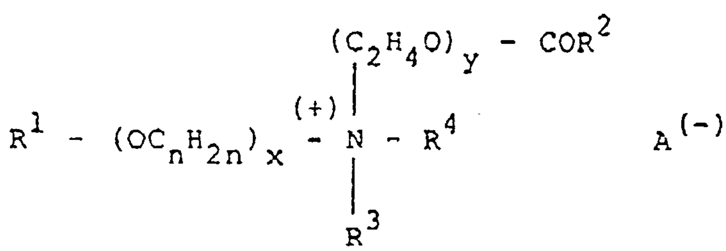

in which $R^1$ is a $C_{8-22}$ alkyl group, $R^2$ is a $C_{7-21}$ alkyl group, $R^3$ is a $C_{1-4}$ alkyl group, a group $-(C_2H_4O)_z$-H or $-(C_2H_4O)_z-COR^2$, $R^4$ is a $C_{1-4}$ alkyl group or a benzyl group, n = 2 or 3, x, y and z are numbers of 1 to 10 and A is a chloride anion, a bromide anion or an anion of the formula $R^5OSO_3^{(-)}$, in which $R^5$ is a $C_{1-4}$ alkyl group.

2. Quaternary ammonium compounds as claimed in claim 1, characterized in that y and z = 1, $R^4$ is a methyl group and A is a chloride anion.

3. A process for the production of the quaternary ammonium compounds of formula I claimed in claim 1 or 2, characterized in that ether amines corresponding to formula II

(II) 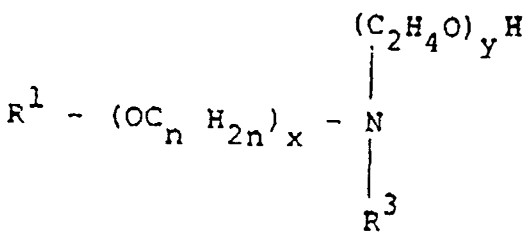

are esterified with a fatty acid of the formula $R^2$-COOH under reaction conditions known per se and the product obtained is quaternized with an alkyl halide or a dialkyl sulfate of the formula $R^4A$.

4. The use of the quaternary ammonium compounds of formula I claimed in claim 1 or 2 as conditioning and softening agents in hair-cosmetic preparations.

**Revendications**

1. Des composés d'ammonium quaternaires selon la formule I

(I) 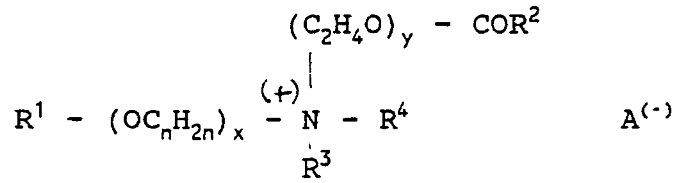

dans laquelle $R^1$ représente un groupe alkyle avec 8 à 22 atomes C, $R^2$ un groupe alkyle avec 7 à 21 atomes C, $R^3$ un groupe alkyle avec 1 à 4 atomes C, un groupe $-(C_2H_4O)_z-H$ ou $-(C_2H_4O)_z-COR^2$, $R^4$ un groupe alkyle avec 1 à 4 atomes C ou un groupe benzyle, n = 2 ou 3, x, y et z des nombres de 1 à 10 et A est un anion chlorure, bromure ou un anion de la formule $R^5OSO_3(-)$, dans laquelle $R^5$ est un groupe alkyle avec 1 à 4 atomes C.

**2.** Des composés ammonium quaternaires selon la revendication 1, caractérisés par le fait que y et z = 1, $R^4$ un groupe méthyle et A un anion chlorure.

**3.** Procédé de fabrication de composés d'ammonium quaternaires selon la formule I conformément à la revendication 1 ou 2, caractérisé par le fait que l'on estérifie des étheramines de la formule II

(II) 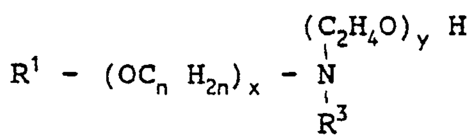

avec un acide gras avec comme formule $R^2$ - COOH, sous des conditions de réaction connues, et quaternise le produit obtenu avec un halogénure d'alkyle ou un sulfate de dialkyle de formule $R^4$ A.

**4.** Utilisation des composés d'ammonium quaternaires selon la formule I conformément à la revendication 1 ou 2 comme agents actifs conditionnant et avivants dans des préparations de cosmétique capillaire.